# NEUE EUROPÄISCHE PATENTSCHRIFT

(11) **EP 1 227 355 B2**
(45) Veröffentlichungstag und Bekanntmachung des Hinweises auf die Entscheidung über den Einspruch: **27.07.2011**
(45) Hinweis auf die Patenterteilung: 11.06.2008
(21) Anmeldenummer: 01121232.1
(22) Anmeldetag: 05.09.2001
(51) Int. Cl.: G02B 21/00, A61B 3/13

(54) **Mikroskop zur Weitwinkelbeobachtung, insbesondere für Augenoperationen**
Microscope for wide angle inspection, especially for eye operations
Microscope pour observation à grand angle, notamment de chirurgie ophthalmologique

(30) Priorität: 23.12.2000 DE 20021955 U
(43) Veröffentlichungstag der Anmeldung: 31.07.2002
(73) Patentinhaber: Oculus Optikgeräte GmbH, 35582 Wetzlar (DE)
(72) Erfinder: Kirchhübel, Rainer, 35614 Asslar (DE); Reiner, Josef, Prof. Dr., 50999 Köln (DE)
(74) Vertreter: Böck, Bernhard

(56) Entgegenhaltungen:
- EP-A- 1 191 381
- WO-A-91/15150
- WO-A2-02/27379
- DE-U- 8 902 035
- DE-U- 9 415 219
- DE-U1- 20 021 955
- US-A- 5 009 487
- US-A- 5 282 085
- US-A- 5 321 447
- US-A- 5 986 801

## Beschreibung

Die Erfindung betrifft ein Mikroskop zur Weitwinkel-Betrachtung eines Auges mit einer zwischen dem Objektiv und dem zu behandelnden Auge befindlichen, ein seitenverkehrtes Bild entwerfenden Optik zur Beobachtung des Augenhintergrundes, insbesondere für Augenoperationen, und mit einer in den Strahlengang des Mikroskops gelegenen, vorzugsweise einschieb- bzw. einschwenkbaren Einrichtung zur Bildumkehrung und -aufrichtung.

Man kann eine Weitwinkel-Beobachtung des Auges in einfacher Weise durch eine dem Auge unmittelbar aufgesetzte Kontaktlinse vornehmen. Stattdessen ist es auch möglich, dass eine gesonderte, von dem Auge beabstandete Optik an dem Mikroskop angebracht ist. Werden dafür asphärische Linsen verwendet, die allein eine ausreichende Weitwinkel-Betrachtung gewährleisten, dann wird genauso wie mit der Kontaktlinse im Mikroskop ein seitenverkehrtes, kopfstehendes Bild erzeugt, welches bei der Diagnostik hingenommen werden kann; bei Augenoperationen, die vorteilhaft mindestens zeitweise ebenfalls bei Weitwinkel-Betrachtung unter Stereobeobachtung durchgeführt werden müssen, kommt eine umgekehrte Stereopsis hinzu, so dass selbst geübten Operateuren eine derartige Kontrolltechnik nicht zugemutet werden kann.

Es ist auch schon bekannt, bei einem derartigen Mikroskop eine Einrichtung zur Bildumkehrung und -aufrichtung vorzusehen, wie das in der DE- Patentschrift 38 26 069 C2 gezeigt ist. Besonders wichtig ist dabei, dass durch die zusätzlichen Bauteile die Bauhöhe des Mikroskops nicht zu stark vergrößert wird, weil der Operateur gleichzeitig die Operation durchführen und durch das Mikroskop schauen muß und deshalb die Entfernung zwischen dem Okular des Mikroskops und dem Auge des Patienten nicht beliebig vergrößert werden kann. Ferner soll aber auch die Einrichtung zur Bildumkehrung und -aufrichtung möglichst schnell in den Strahlengang des Mikroskops eingebracht und wieder daraus entfernt werden können, damit sowohl im Vorderabschnitt des Auges als auch im Augenhintergrund gearbeitet werden kann, ohne das Mikroskop wechseln zu müssen.

Die Erfindung hat sich deshalb die Aufgabe gestellt, ein Mikroskop der eingangs näher bezeichneten Art so auszubilden, dass seine Bauhöhe auch dann nicht wesentlich vergrößert werden muß, wenn es wahlweise mit einer Einrichtung zur Bildumkehrung und -aufrichtung betrieben werden kann.

Erfindungsgemäß wird ein Mikroskop gemäß Anspruch 1 bereitgestellt. Die erfindungsgemäße Anordnung macht sich den zwischen dem Objektiv und dem zu behandelnden Auge vorhandenen Raum zunutze, so dass insgesamt die Bauhöhe des Mikroskops auch dann erhalten bleibt, wenn in dem Okular ein seitenrichtiges und aufrechtstehendes Bild erzeugt wird. Dabei spielt es keine Rolle, in welcher Weise die Weitwinkel-Betrachtung produziert wird: die Optik zur Beobachtung des Augenhintergrundes kann sowohl an dem Halter für das Prismensystem angebracht als auch unmittelbar auf das Auge aufsetzbar ausgebildet sein. Auf diese Weise entsteht sofort nach dem Einschwenken bzw. Einschieben der Einrichtung ein seitenrichtiges und aufrechtstehendes Bild, das nicht erst durch eine weitere Betätigung hergestellt werden muß, die dazu sonst erforderliche Hand- oder Fußbetätigung entfällt, was gerade bei einer Augenoperation von großem Vorteil ist.

Die Einrichtung zur Bildumkehrung und -aufrichtung kann in den Bereich zwischen dem Objektiv und dem Auge eingeschoben werden. Wesentlich einfacher ist es aber, wenn der Halter um eine an der Unterseite des

Mikroskops an diesem angeordnete Schwenkachse drehbar ist, so dass nur wenige Bauteile erforderlich sind, um die Einrichtung aus einer Betriebsbereitschafts-Stellung in den Strahlengang des Mikroskops einzuschwenken. Das Prismensystem ist dabei am besten in einem geschlossenen Gehäuse angeordnet, das mit Durchbrechungen für den Strahlengang versehen ist. Zwischen dem Prismensystem und dem Objektiv kann noch eine nach dem Einschieben bzw. Einschwenken des Prismensystems in den Strahlengang des Mikroskops dem Objektiv unmittelbar benachbarte Abbildungsoptik zur Anpassung des Strahlenganges vorgesehen sein, am besten in der dem Objektiv benachbarten Durchbrechung des Gehäuses. Im übrigen ist es zweckmäßig, wenn die Schwenkachse für den Halter etwa waagerecht an dem Mikroskop vorgesehen ist.

Die Optik zur (Weitwinkel-) Beobachtung des Augenhintergrundes kann aus einem längs des Strahlenganges beweglich angeordneten Linsensystem bestehen. Der Abstand dieses Linsensystems zu dem Auge kann während der Arbeit des Operateurs von diesem unverändert belassen werden, weil zum Fokussieren in dem Strahlengang zwischen der Optik zur Beobachtung des Augenhintergrundes und dem Prismensystem eine längs des Strahlenganges und relativ zu dem Prismensystem bewegliche Optik vorgesehen ist; dazu genügt bereits eine einzelne optische Linse, die ihrerseits fokussierbar ist.

Es ist vorteilhaft, wenn zum Fokussieren beide Optiken zur Weitwinkel-Beobachtung und/oder zur Anpassung der Zwischenabbildung mittels handoder elektromotorisch angetriebener Spindeltriebe betätigbar sind. So kann etwa die Optik zur Beobachtung des Augenhintergrundes mittels eines an dem Halter befestigten ersten Spindeltriebes längs des Strahlenganges bewegbar sein, am einfachsten in der Weise, dass die Optik an einer Traverse gehaltert ist, welche auf einem an dem Halter angebrachten und zu dem ersten Spindeltrieb sich parallel erstreckenden Führungsstift längsbeweglich geführt ist, wobei an dem Führungsstift ein erster Drehknopf für den ersten Spindeltrieb gelagert ist. Die gesamte Verstell-Mechanik für die Optik ist auf diese Weise mit dem Halter und damit auch mit dem Prismensystem verbunden und bleibt stets genau justiert.

In ähnlicher Weise kann mit der Optik zur Anpassung der Zwischenbeobachtung verfahren werden, wenn diese mittels eines an dem Führungsstift befestigten zweiten Spindeltriebes längs des Strahlenganges bewegbar ist, wobei hierzu ein zweiter Drehknopf dient.

Das Prismensystem kann, beispielsweise, als Reflexionsprisma nach Uppendahl oder Schmidtpechan ausgebildet sein.

Die Drehknöpfe für die Spindeltriebe können von Hand betätigbar sein; in vorteilhafter Weise sind sie aber mittels eines elektrischen Antriebes betätigbar, wobei ein solcher Antrieb beispielsweise einen vorzugsweise an dem Halter befindlichen Elektromotor aufweist, dessen Abtrieb über eine biegsame Welle mit dem Drehknopf rotatorisch koppelbar ist, so dass die Schaltung gegebenenfalls durch einen fußbetätigten Schalter vorgenommen werden, so dass der Operateur seine Hände frei behält, wenn nachfokussiert werden muß.

Die Anordnung der Einrichtung zur Bildumkehrung und - aufrichtung ist deshalb recht universell an Mikroskopen ganz unterschiedlicher Bauart einsetzbar; vorhandene Mikroskope können ohne viel Aufwand entsprechend nachgerüstet und damit den Anforderungen während der operativen Versorgung besser angepaßt werden. Die Einrichtung kann aber auch sehr schnell aus dem Strahlengang des Mikroskops wieder entfernt werden, ohne dass der Operateur seine Arbeit am Auge eines Patienten unterbrechen oder dazu eine Hilfskraft hinzugezogen werden müßte.

Die Erfindung wird nachstehend an Hand der Zeichnungen noch weiter erläutert. Es zeigen
- Fig. 1: ein Mikroskop mit einer Optik zur Weitwinkel-Beobachtung und einem Prismensystem nach Uppendahl in der Seitenansicht, das nicht in den Schutzumfang von Anspruch 1 fällt.
- Fig. 2: ein Mikroskop entsprechend Fig. 1, jedoch mit einem Prismensystem nach Schmidtpechan,
- Fig. 3: bzw. Fig. 4 ein Mikroskop entsprechend Fig. 1 bzw. Fig. 2 mit jeweils einem elektrischen Antrieb für die Optik zur Weitwinkel-Beobachtung,
- Fig. 5: ein Mikroskop entsprechend Fig. 1 mit einer gegenüber Fig. 3 veränderten Optik zur Weitwinkel-Beobachtung,
- Fig. 6: ein Mikroskop entsprechend Fig. 2 mit einer gegenüber Fig. 4 veränderten Optik zur Weitwinkel-Beobachtung,
- Fig. 7: bzw. Fig. 8 ein Mikroskop entsprechend Fig. 1 bzw. Fig. 2 mit einer von Hand antreibbaren Optik zur Anpassung der Zwischenabbildung,
- Fig. 9: bzw. 10. ein Mikroskop entsprechend Fig. 7 bzw. Fig. 8 mit einer elektrisch antreibbaren Optik zur Anpassung der Zwischenabbildung,

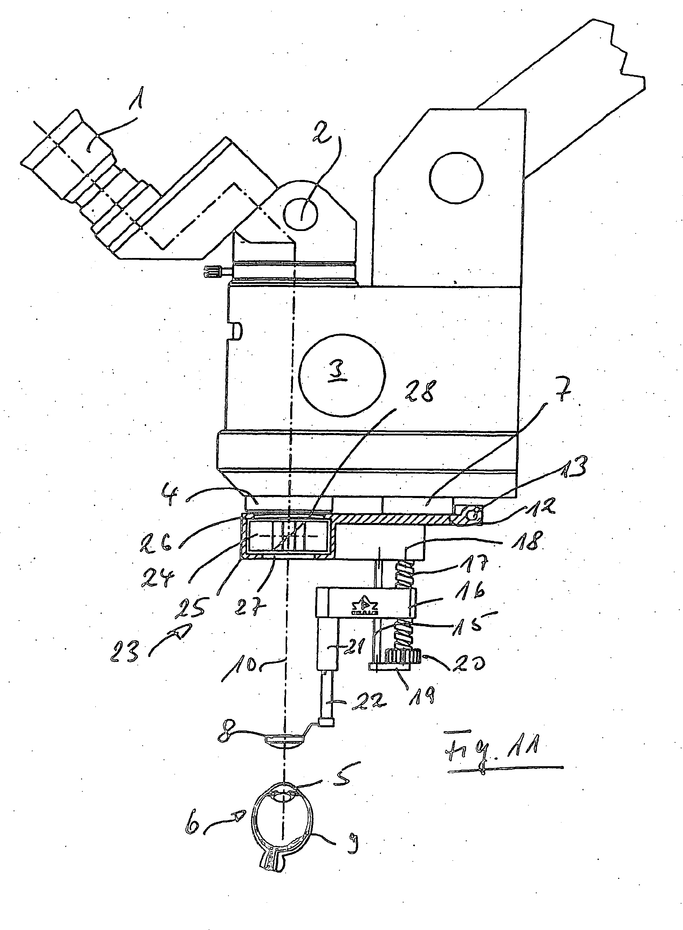

- Fig. 11: ein Mikroskop entsprechend den Fig. 1 bis 5, mit einem Porroprisma 2. Art,

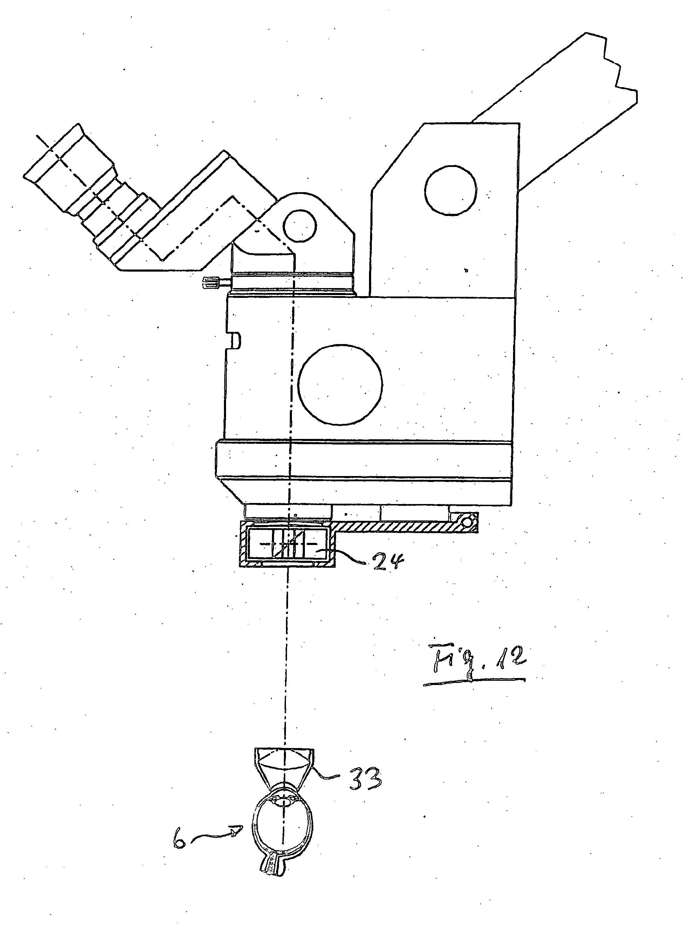

- Fig. 12: ein Mikroskop entsprechend Fig. 6, jedoch mit einem Porroprisma 2. Art,

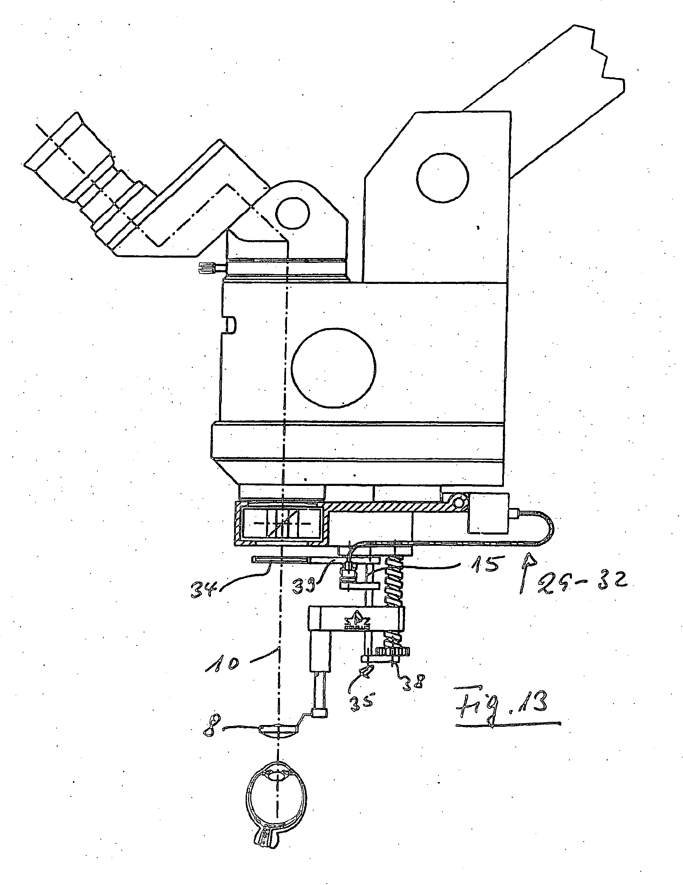

- Fig. 13: ein Mikroskop entsprechend Fig. 10, jedoch mit einem Porroprisma 2. Art,
- Fig. 14: den Strahlengang in einem Mikroskop, entsprechend den Fig. 1 bis 13,
- Fig. 15: den gleichen Strahlengang wie in Fig. 14, jedoch unter Verwendung von vier weiteren Prismensystemen und
- Fig. 16: den gleichen Strahlengang wie in Fig. 15, jedoch unter Verwendung zusätzlicher Zerstreuungs- und Sammellinsen,
sämtlich in schematischer, vereinfachter Darstellung.

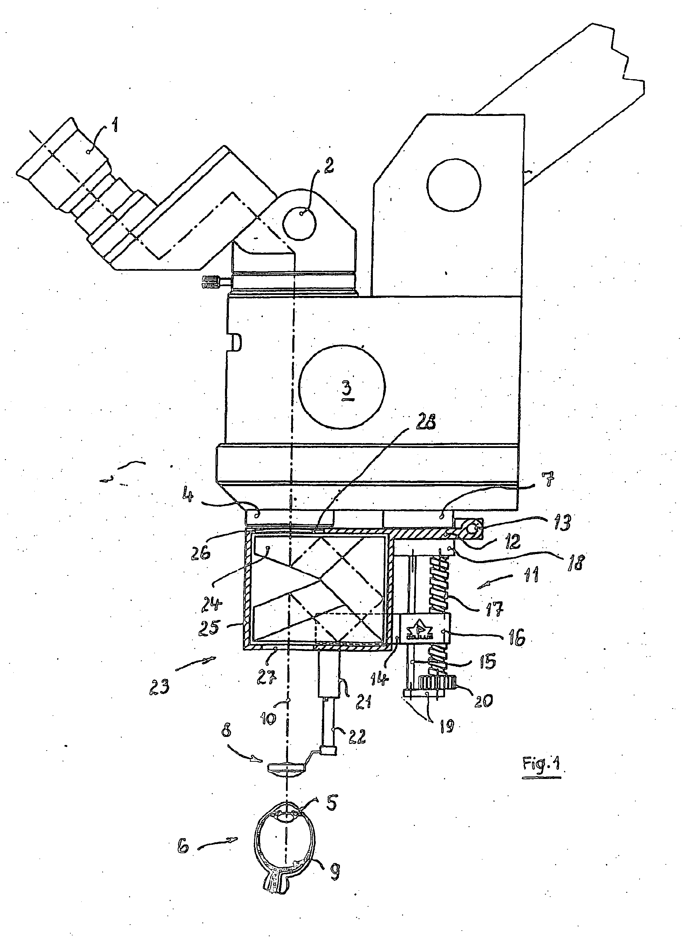

An einem Mikroskop befindet sich zunächst entsprechend Fig.1-10 ein Okular 1, welches um eine Achse 2 schwenkbar ist, so dass, beispielsweise bei einer Augenoperation, der Operateur das Okular 1 seiner Körperhaltung beim Operieren optimal anpassen kann. Ferner ist an dem Mikroskop eine Verstellung 3 zur Änderung des Vergrößerungsmaßstabes vorgesehen. Ein Objektiv 4 ermöglicht zunächst die Beobachtung des Vorderabschnittes des Auges 5 an einem Auge 6.

An einem Ansatz 7 des Mikroskops in der Nachbarschaft des Objektivs 4 ist eine Optik 8 zur Beobachtung des Augenhintergrundes 9 gehaltert, die in den Strahlengang 10 des Mikroskops einschwenkbar und mittels eines (ersten) Spindeltrieb 11 in Richtung des Strahlenganges 10 bewegbar ist. Diese Optik 8, hier eine einfache Beobachtungslinse, ist an einem Halter 12 angebracht, welcher um eine an dem Ansatz 7 ortsfeste Schwenkachse 13 schwenkbar ist. Die Optik 8 ist an einer (ersten) Traverse 14 befestigt, die auf einem Führungsstift 15 parallel zu dem Strahlengang 10 unterhalb des Objektivs 4 bewegbar geführt ist. Die Traverse 14 wird durch einen nicht dargestellten Mitnehmer, der in die Gewindespindel 17 eingreift, bewegt. Der Führungsstift 15 und die Gewindespindel 17 sind einerseits in einem gemeinsamen, an dem Halter 12 arretierten Lagerstück 18 und andererseits in einer (ersten) Verbindungslasche 19 befestigt. Die Gewindespindel 17 ist um ihre Achse drehbar gelagert. In Verbindung mit der Gewindespindel 17 ist ein (erster) Drehknopf 20 vorgesehen, mit dessen Hilfe der Spindelabtrieb 11 in Gang setzbar und die Optik 8 längs des Strahlenganges 10 bewegbar ist. Die Optik 8 ist an dem Haltebolzen 22 befestigt, der federnd in einer Führung 21 gehalten ist. Der komplette Spindeltrieb ist am Lagerstück 18 vom Halter 12 trennbar, so dass dieser sterilisierbar ist.

Eine mit der Optik 8 durch den gemeinsamen Halter 12 verbundene Einrichtung 23 zur Bildumkehrung und -aufrichtung besteht aus einem Porroprismensystem 2. Art 24 und einem das Prismensystem 24 aufnehmendem Gehäuse 25; das Gehäuse 25 kann mit dem Halter 12 und dem Lagerstück 18 einstückig ausgeführt sein. Vorteilhaft ist das Lagerstück 18 vom Halter 12 abnehmbar ausgebildet.

Je eine Durchbrechung 26,27 in dem Gehäuse 25 gestatten den Durchgang des Strahlenganges 10 durch das Prismensystem 24. Dem Eingang des Strahlenganges 10 in das Prismensystem 24 vorgeschaltet ist in der dem Objektiv 4 benachbarten Durchbrechung 26 eine Abbildungsoptik 28 zur Anpassung des wegen des Prismensystems 24 erheblich verlängerten Strahlenganges 10.

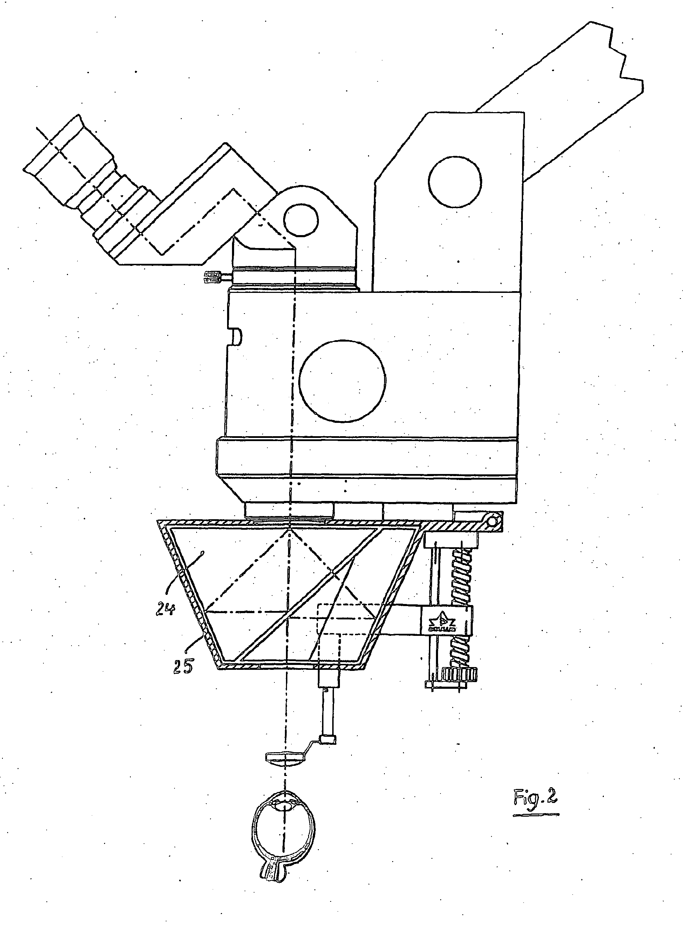

Entsprechend dem in der Fig.2 verwendeten Prismensystem 24 nach Schmidtpechan ist dort das Gehäuse 25 etwas anders ausgeformt, im übrigen unterscheidet sich die Anordnung von der der Fig. 1 aber nicht.

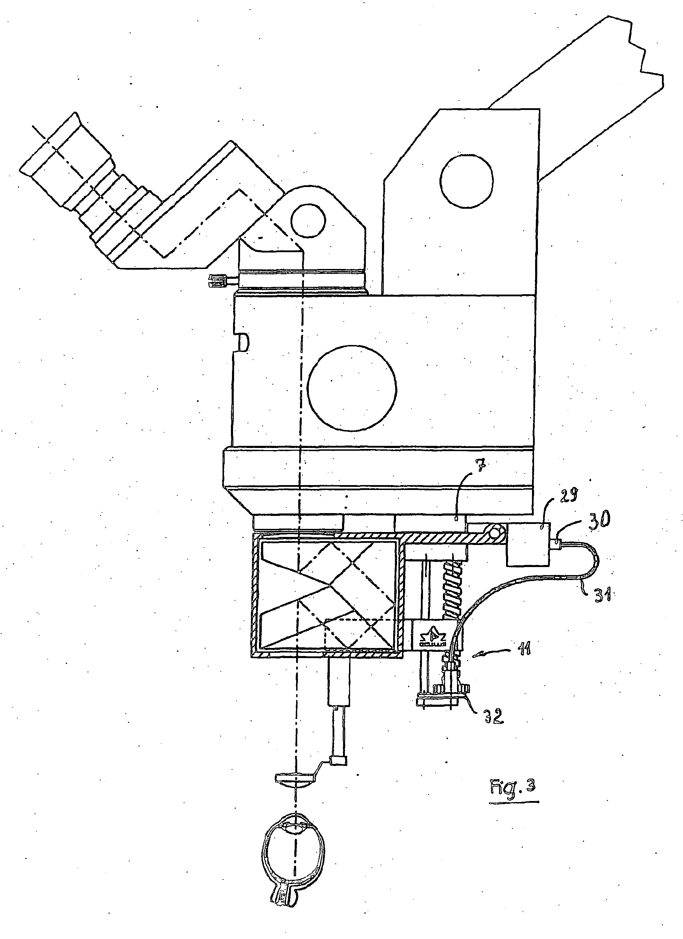

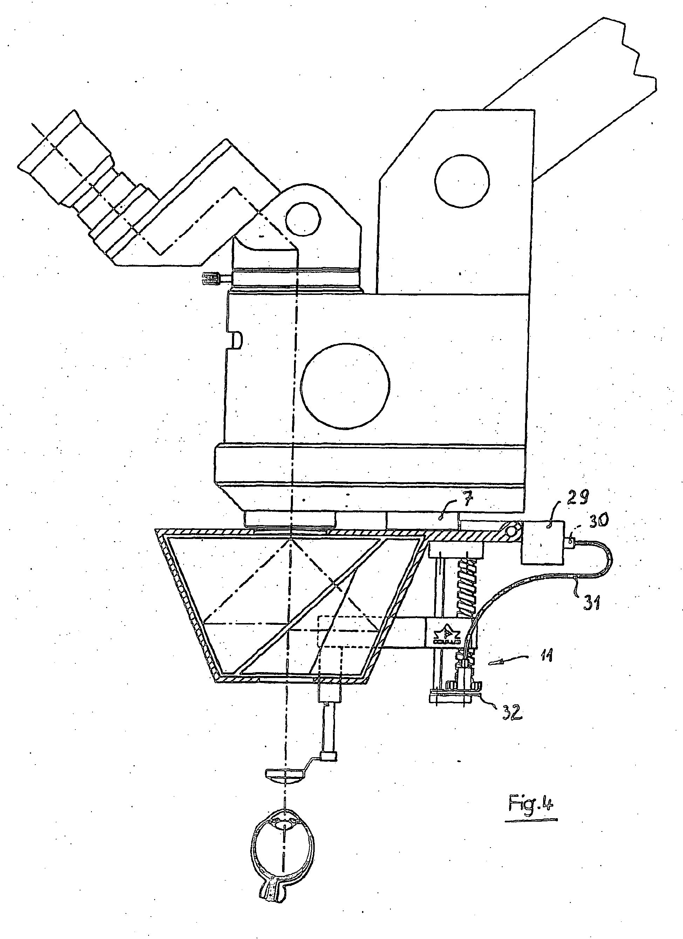

In den Fig.3,4 ist der Spindeltrieb 11 mit einem elektromotorischen Antrieb ausgerüstet. Dazu ist an dem Ansatz 7 ein Elektromotor 29 vorgesehen, von dessen Abtrieb aus über eine mittels geeigneter Kupplung 30 anschließbare biegsame Welle 31 und einem Riementrieb 32 die Gewindespindel 17 in Drehung versetzt werden kann. Der Elektromotor kann auch an anderer Stelle des Systems angebracht werden. Es genügt dementsprechend, den Elektromotor 29 von einem Fußschalter aus zu schalten, um die Optik 8 längs des Strahlenganges 10 zu bewegen; beispielsweise kann ein Operateur dementsprechend fokussieren, ohne seine Operationsinstrumente aus der Hand legen und damit die laufende Operation unterbrechen zu müssen.

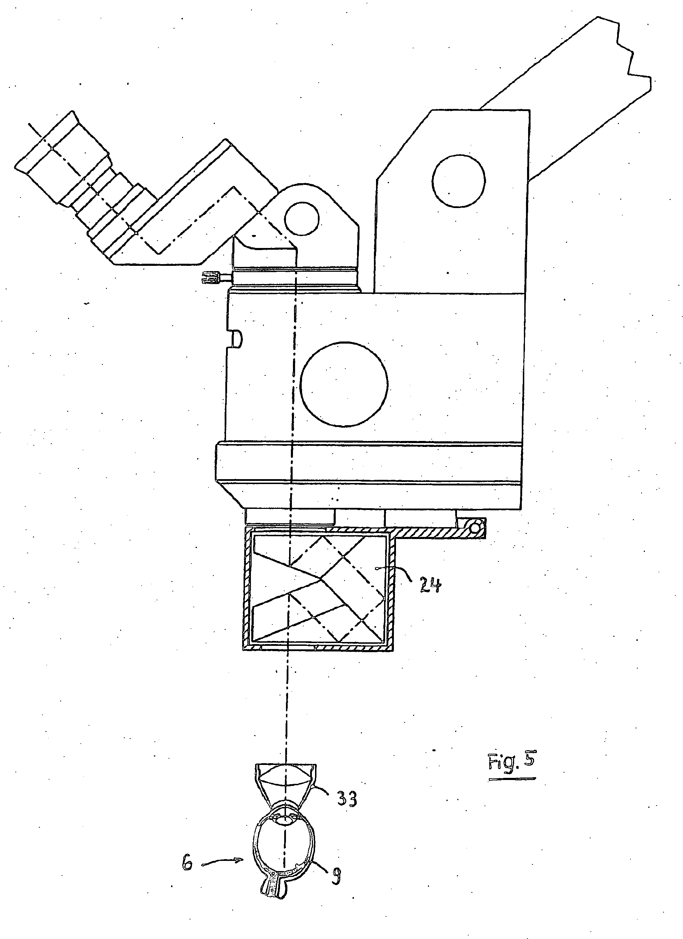

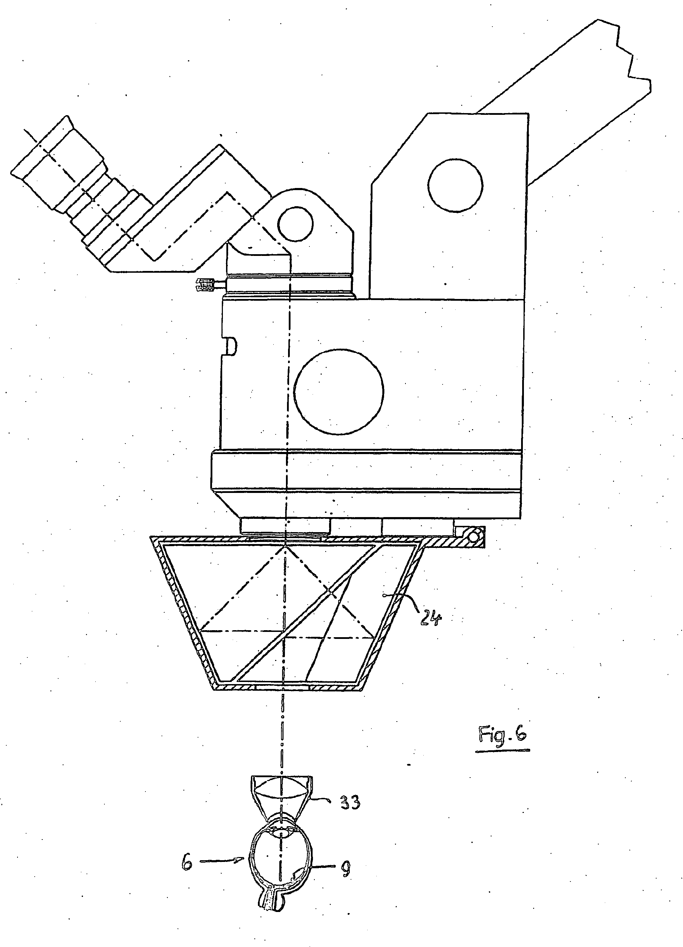

Anstelle einer Nonkontakt-Beobachtung wie in den Anordnungen der Fig.1-4 mittels der Optik 8 kann entsprechend Fig. 5,6 auf eine asphärische, unmittelbar dem Auge 6 aufsetzende Optik 33 zurückgegriffen werden, die in ähnlicher Weise wie die Optik 8 zunächst eine seitenverkehrte und kopfstehende Abbildung des Augenhintergrundes 9 liefert, die wiederum von dem Prismensystem 24 nutzbar gemacht wird.

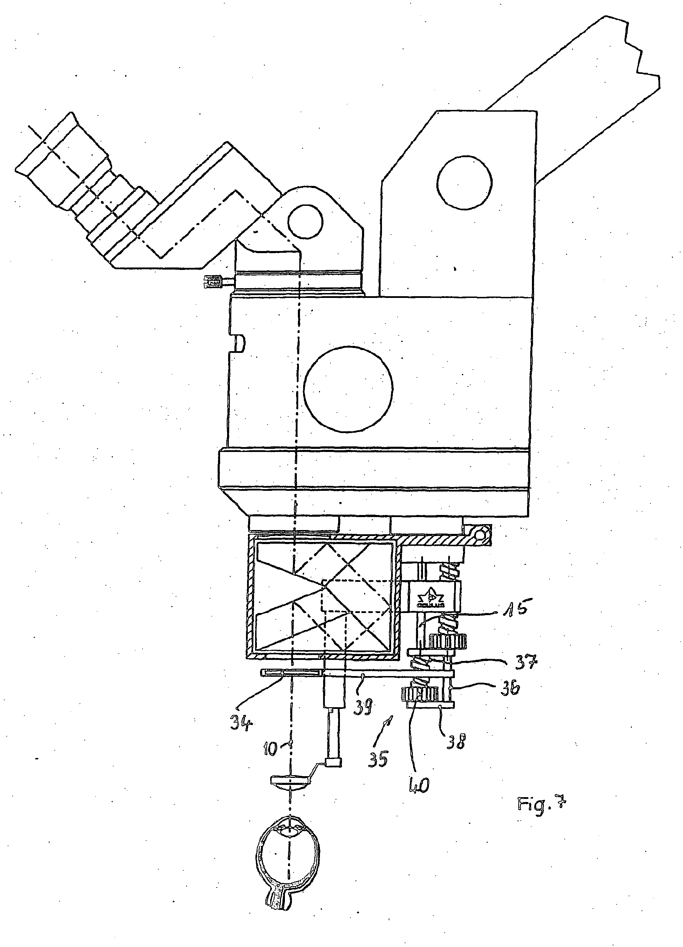

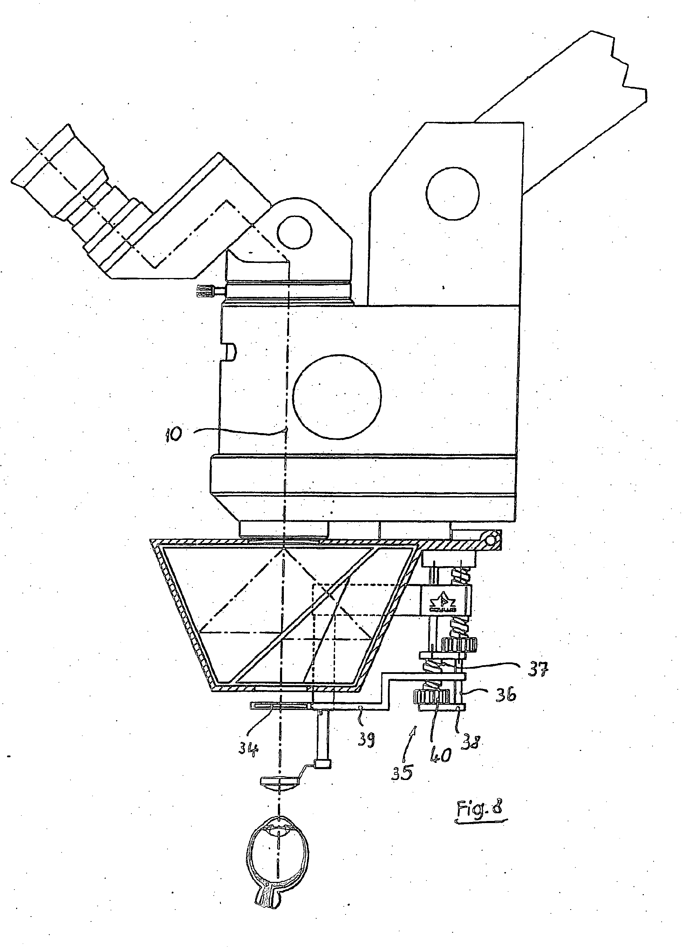

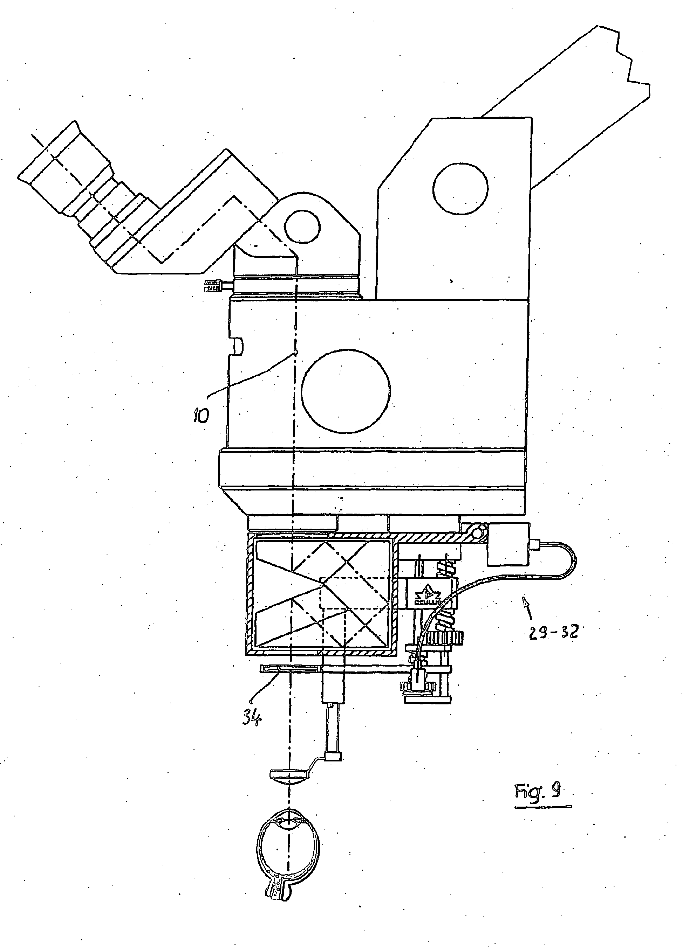

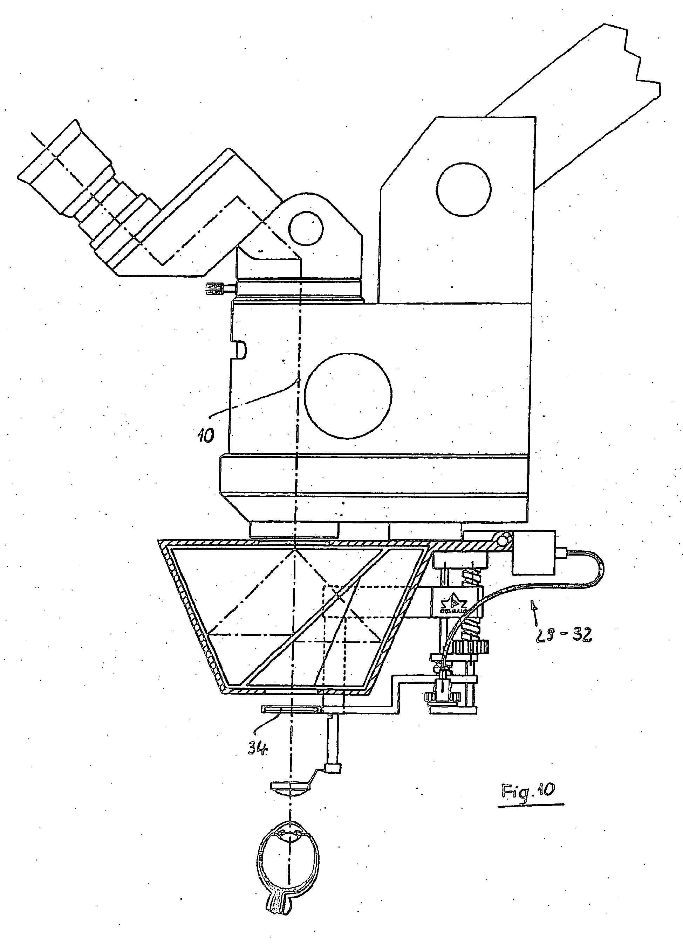

Eine Möglichkeit, die Optik 8 zu fokussieren, ohne sie überhaupt bewegen zu müssen, bietet gemäß der Erfindung eine längs des Strahlenganges 10 verschiebbare Optik 34 zur Anpassung der Zwischenabbildung entsprechend Fig.7-10. In ähnlicher Weise wie die Optik 8 zur Beobachtung des Augenhintergrundes 9 ist für die Optik 34 - hier eine einfache Linse - ein (zweiter) Spindeltrieb 35 installiert, wobei je ein weiterer Führungsstift 36 und eine Gewindespindel 37 auf der von dem ersten Spindeltrieb 11 abgewandten Seite der ersten Verbindungslasche 19 befestigt und an ihrem jeweils anderen Ende von einer (zweiten) Verbindungslasche 38 zusammengehalten werden. Die Optik 34 ist in einer (zweiten) Traverse 39 gehaltert, die ganz ebenso wie die erste Traverse 14 einen Mitnehmer enthält, welche von der rotierenden Gewindespindel 37 in Richtung des Strahlenganges 10 bewegt wird, wenn ein entsprechender, an der Gewindespindel 37 gehalterter (zweiter) Drehknopf 40 betätigt wird (Fig.7,8). Der Spindeltrieb 35 kann aber auch entsprechend Fig.9,10 in ähnlicher Weise wie der Spindeltrieb 11 mittel des elektrischen Antriebes 29-32 betätigt werden.

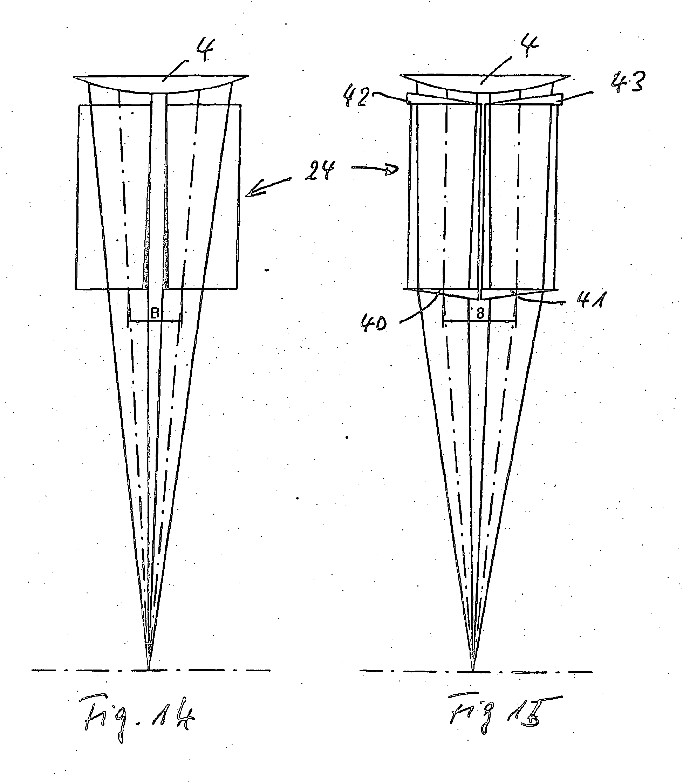

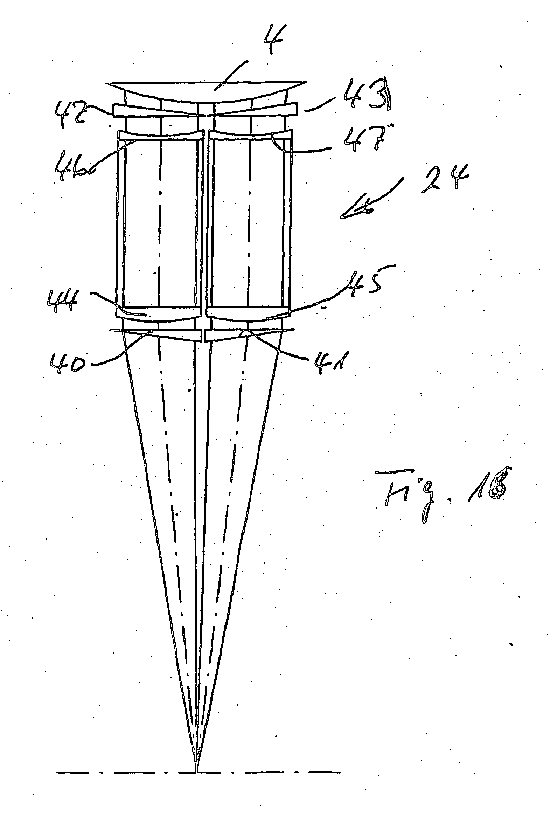

Die Fig. 14 zeigt den Strahlengang unterhalb des Mikroskops, wobei dieser Strahlengang durch die Verwendung von vier Prismen, wie sie Fig. 15, 16 zeigen, verbessert worden ist. Insbesondere ergibt sich hierdurch eine Vergrößerung der stereoskopischen Basis, wobei gleichfalls Aberrationen wegfallen. Der Vorteil dieser Anordnung besteht darin, dass keine Abschattungen auftreten können, und somit ein besseres stereoskopisches Sehen gewährleistet ist. Die verwendeten Prismen sind gleich stark ausgebildet, wobei die Basis der unten liegenden Prismen 40 und 41 zueinander zugerichtet ist, während die dem Objektiv 4 nächst liegenden Prismen 42 und 43 ihre Basis nach außen gerichtet haben. Die mit dem Pfeil B eingezeichnete stereoskopische Breite wird hierdurch wesentlich verbessert. Die Prismen haben z.B. bei einer Objektivbrennweite von 200 mm vorteilhaft 5 pdpt (Prismendioptrin).

In Fig. 16 sind die gleichen Prismen 40 bis 43 unterhalb bzw. oberhalb des Prismensystems 24 angeordnet, wobei weiterhin zwischen den Prismen 40, 41 bzw. 42, 43 jeweils eine Sammellinse 44, 45 bzw. eine Zerstreuungslinse 46 bzw. 47 angeordnet sind. Hierdurch wird die Abbildung weiter vervollkommnet, so dass ein paralleler Strahlenverlauf der Öffnungsstrahlen im Prismensystem erhalten wird. Der Arbeitsabstand von der Eintrittsfläche wird beibehalten und entspricht der Brennweite der großen Linse 4.

### Bezugszeichenliste

- 1: Okular
- 2: Achse
- 3: Verstellung
- 4: Objektiv
- 5: Vorderabschnitt des Auges
- 6: Auge
- 7: Ansatz
- 8: Optik (zur Beobachtung des Augenhintergrundes 9)
- 9: Hinterabschnitt des Auges
- 10: Strahlengang
- 11: (erster) Spindeltrieb
- 12: Halter
- 13: Schwenkachse
- 14: (erste) Traverse
- 15: Führungsstift
- 16: Mitnehmer
- 17: Gewindespindel
- 18: Lagerstück
- 19: (erste) Verbindungslasche
- 20: (erster) Drehknopf
- 21: Führung
- 22: Haltebolzen
- 23: Einrichtung (zur Bildumkehr und -aufrichtung)
- 24: Prismensystem
- 25: Gehäuse
- 26: Durchbrechung
- 27: Durchbrechung
- 28: Abbildungsoptik
- 29: Elektromotor
- 30: Kupplung
- 31: (biegsame) Welle
- 32: Riementrieb
- 29-32: elektrischer Antrieb
- 33: Optik (das Auge kontaktierend)
- 34: Optik (zur Anpassung der Zwischenabbildung)
- 35: (zweiter) Spindeltrieb
- 36: (zweiter) Führungsstift
- 37: (zweite) Gewindespindel
- 38: (zweite) Verbindungslasche
- 39: (zweite) Traverse
- 40: (zweiter) Drehknopf
- 40-43: Prismen
- 44/45: Sammellinsen
- 46/47: Zerstreuungslinsen

## Patentansprüche

1. Mikroskop zur Weitwinkel-Betrachtung eines Auges (6) mit einer zwischen dem Objektiv (4) und dem zu behandelnden Auge (6) befindlichen, ein seitenverkehrtes Bild entwerfenden Optik (8,33) zur Beobachtung des Augenhintergrundes (9), insbesondere für Augenoperationen, und mit einer in den Strahlengang (10) des Mikroskops gelegenen, einschieb- bzw. einschwenkbaren Einrichtung (23) zur Bildumkehrung und -aufrichtung, wobei die Einrichtung (23) zur Bildumkehrung und -aufrichtung aus einem in der Höhe niedrig bauenden reflektierenden System, einem Prismensystem (24) besteht, das von einem an dem Mikroskop befestigten Halter (12) getragen und so in den Strahlengang (10) des Mikroskops zwischen dem Objektiv (4) und dem zu behandelnden Auge (6) einschieb- bzw. einschwenkbar ist, dass sich das Prismensystem (24) unmittelbar vor dem Objektiv (4), mit Abstand zu dem Auge (6), befindet, wobei jedoch zwischen dem Prismensystem (24) und dem Objektiv (4) eine nach dem Einschieben bzw. Einschwenken des Prismensystems (24) in den Strahlengang (10) des Mikroskops dem Objektiv (4) unmittelbar benachbarte Abbildungsoptik (28) zur Anpassung des Strahlenganges (10) vorgesehen ist,
wobei
in dem Strahlengang (10) zwischen der Optik (8) zur Beobachtung des Augenhintergrundes (9) und dem Prismensystem (24) eine längs des Strahlenganges (10) und relativ zu dem Prismensystem (24) bewegliche Optik (34) zur Anpassung der Zwischenabbildung vorgesehen ist, so dass die Optik (8) fokussiert werden kann, ohne sie zu bewegen wobei vor und hinter jedem Prismensystem (24) jeweils zwei, im wesentlichen in einer Ebene liegende, einen Strahlengang überdeckende Prismen (40-43) angeordnet sind, wobei die Prismenbasis der jeweils in etwa in einer Ebene liegende Prismen (40-43) entgegengesetzt angeordnet sind, wobei die Prismenbasis der dem Objektiv nächstliegenden Prismen (42, 43) jeweils voneinander ab- und die der anderen Prismen (40, 41) aufeinander zugewandt sind.

2. Mikroskop nach Anspruch 1,
**dadurch gekennzeichnet,**
**dass** die Optik (8) zur Beobachtung des Augenhintergrundes (9) an dem Halter (12) angebracht ist.

3. Mikroskop nach einem der Ansprüche 1 oder 2,
**dadurch gekennzeichnet,**
**dass** der Halter (12) um eine an der Unterseite des Mikroskops an diesem angeordnete Schwenkachse (13) drehbar oder in einer Führung verschiebbar ist.

4. Mikroskop nach einem der Ansprüche 1 bis 3,
**dadurch gekennzeichnet,**
**dass** das Prismensystem (24) in einem geschlossenen Gehäuse (25) angeordnet ist, das mit Durchbrechungen (26,27) für den Strahlengang (10) versehen ist.

5. Mikroskop nach Anspruch 3,
**dadurch gekennzeichnet,**
**dass** die Schwenkachse (13) etwa waagerecht oder senkrecht an dem Mikroskop vorgesehen ist.

6. Mikroskop nach einem der Ansprüche 1 bis 5,
**dadurch gekennzeichnet,**
**dass** die Optik (8) zur Beobachtung des Augenhintergrundes (9) aus einem längs des Strahlenganges (10) beweglich angeordneten Linsensystem besteht.

7. Mikroskop nach einem der Ansprüche 1 bis 6,
**dadurch gekennzeichnet,**
**dass** die Optiken (8,34) zur Weitwinkelbeobachtung und/oder zur Zwischenabbildung mittels hand- oder elektromotorisch angetriebener Spindeltriebe (11,35; 29-32) betätigbar sind.

8. Mikroskop nach einem der Ansprüche 1 bis 7,
**dadurch gekennzeichnet,**
**dass** als Prismensystem (24) ein Porroprismensystem 2. Art oder ein Reflexionsprisma nach Uppendahl dient.

9. Mikroskop nach einem der Ansprüche 1 bis 7,
**dadurch gekennzeichnet,**
**dass** als Prismensystem (24) ein Reflexionsprisma nach Schmidt Pechan dient.

10. Mikroskop nach einem der Ansprüche 1 bis 9,
**dadurch gekennzeichnet,**
**dass** der Halter (12) mittels eines ersten Spindeltriebes (11) längs des Strahlenganges (10) bewegbar ist.

11. Mikroskop nach Anspruch 10,
**dadurch gekennzeichnet,**
**dass** die Optik (8) an einer Traverse (14) gehaltert ist, welche auf einem an dem Halter (12) angebrachten und zu dem ersten Spindeltrieb (11) sich parallel erstreckenden Führungsstift (15) längsbeweglich geführt ist, wobei an der Gewindespindel (17) ein erster Drehknopf (20) für den ersten Spindeltrieb (11) gelagert ist.

12. Mikroskop nach Anspruch 11,
**dadurch gekennzeichnet,**
**dass** die Optik (34) zur Anpassung der Zwischenbeobachtung mittels eines an dem Führungsstift (15) befestigten zweiten Spindeltriebes (35) längs des Strahlenganges (10) bewegbar ist, wobei der erste Führungsstift (15) über eine Verbindungslasche (19) mit dem zweiten Führungsstift (36) verbunden ist und dass ein zweiter Drehknopf (40) zum Antrieb des zweiten Spindeltriebes (35) vorgesehen ist.

13. Mikroskop nach Anspruch 11 oder 12,
**dadurch gekennzeichnet,**
**dass** die Drehknöpfe (20,40) von Hand betätigbar sind.

14. Mikroskop nach Anspruch 11 oder 12,
**dadurch gekennzeichnet,**
**dass** zumindest einer der Drehknöpfe (20,40) mittels eines elektrischen Antriebes (29-32) betätigbar ist.

15. Mikroskop nach Anspruch 14,
**dadurch gekennzeichnet,**
**dass** der Antrieb (29-32) einen vorzugsweise an dem Halter (12) befindlichen Elektromotor (29) aufweist, dessen Abtrieb über eine biegsame Welle (31) mit dem Drehknopf (20,40) rotatorisch koppelbar ist.

16. Mikroskop nach einem der Ansprüche 1 bis 15,
**dadurch gekennzeichnet,**
**dass** das Prismensystem (24) aus zwei Bildumkehrungs- und - aufrichtungseinheiten besteht.

17. Mikroskop nach einem der Ansprüche 1 bis 16,
**dadurch gekennzeichnet,**
**dass** zwischen jedem Prisma (40-43) und dem Prismensystem (24) eine Sammel- oder Zerstreuungslinse (44-47) angeordnet ist und dass die Zerstreuungslinsen (46, 47) dem Objektiv (4) des Mikroskops benachbart sind.

## Claims

1. A microscope for the wide-angle viewing of an eye (6) with an optical device (8, 33)situated between the lens (4) and the eye (6) which is to be treated and which creates a reversed image, for observing the fundus of the eye (9), in particular for eye operations, and with a device (23) for image reversion and image erection which is situated and able to be moved or swung into the beam path (10) of the microscope, in which the device (23) for image reversion and image erection consists of a reflecting system having a small height, a prism system (24) which is carried by a holder (12) fastened on the microscope and is able to be moved or swung into the beam path (10) of the microscope between the lens (4) and the eye (6) which is to be treated such that the prism system (24) is situated directly in front of the lens (4), at a distance from the eye (6), in which, however, between the prism system (24) and the lens (4) a projection lens (28) is provided for adjusting the beam path (10), said projection lens being directly adjacent to the lens (4) after the prism system (24) has been moved or swung into the beam path (10) of the microscope, wherein in the beam path (10) between the optical device (8) for observing the fundus of the eye (9) and the prism system (24), an optical device (34) for adjusting the intermediate image is provided which is movable along the beam path (10) and relative to the prism system (24), so that the optical device (8) can be focussed without moving it, wherein two prisms (40-43) lying substantially in one plane and superposing a beam path are respectively arranged in front and behind each prism system (24), in which the prism bases of the prisms (40-43) respectively lying approximately in one plane are oppositely arranged, in which the prism bases of the prisms (42, 43) lying closest to the lens respectively face away from each other and those of the other prisms (40, 41) face each other.

2. The microscope according to Claim 1,
**characterized in that**
the optical device (8) for observing the fundus of the eye (9) is mounted on the holder (12).

3. The microscope according to any of Claims 1 or 2,
**characterized in that**
the holder (12) is rotatable about a swivel axis (13) arranged on the underside of the microscope on the latter or is movable in a guideway.

4. The microscope according to any of Claims 1 to 3,
**characterized in that**
the prism system (24) is arranged in a closed housing (25) which is provided with openings (26, 27) for the beam path (10).

5. The microscope according to Claim 3,
**characterized in that**
the swivel axis (13) is provided approximately horizontally or perpendicularly on the microscope.

6. The microscope according to any of Claims 1 to 5,
**characterized in that**
the optical device (8) for observing the fundus of the eye (9) consists of a lens system movably arranged along the beam path (10).

7. The microscope according to any of Claims 1 to 6,
**characterized in that**
the optical devices (8, 34) for wide angle observation and/or for the intermediate image are able to be actuated by means of manually or electromotively driven spindle drives (11, 35; 29-32).

8. The microscope according to any of Claims 1 to 7,
**characterized in that**
a Porro prism system of the second type or an Uppendahl reflecting prism serves as the prism system (24).

9. The microscope according to any of Claims 1 to 7,
**characterized in that**
a Schmidt-Pechan reflecting prism serves as the prism system (24).

10. The microscope according to any of Claims 1 to 9,
**characterized in that**
the holder (12) is movable along the beam path (10) by means of a first spindle drive (11).

11. The microscope according to Claim 10,
**characterized in that**
the optical device (8) is supported on a carriage (14) which is guided so as to be longitudinally movable on a guide pin (15) mounted on the holder (12) and extending parallel to the first spindle drive (11), in which a first rotary knob (20) for the first spindle drive (11) is mounted on the threaded spindle (17).

12. The microscope according to Claim 11,
**characterized in that**
the optical device (34) for adjusting the intermediate observation is movable along the beam path (10) by means of a second spindle drive (35) which is fastened to the guide pin (15), in which the first guide pin (15) is connected to the second guide pin (36) by a connecting plate (19) and a second rotary knob (40) is provided for driving the second spindle drive (35).

13. The microscope according to Claim 11 or 12,
**characterized in that**
the rotary knobs (20, 40) are able to be actuated manually.

14. The microscope according to Claim 11 or 12,
**characterized in that**
at least one of the rotary knobs (20, 40) is able to be actuated by means of an electric drive (29-32).

15. The microscope according to Claim 14,
**characterized in that**
the drive (29-32) has an electric motor (29) which is preferably situated on the holder (12), the output of which motor is able to be rotationally coupled with the rotary knob (20, 40) by a flexible shaft (31).

16. The microscope according to any of Claims 1 to 15,
**characterized in that**
the prism system (24) consists of two image-reversing and image-erecting units.

17. The microscope according to any of Claims 1 to 16,
**characterized in that**
a focussing or dispersing lens (44-47) is arranged between each prism (40-43) and the prism system (24) and that the dispersing lenses (46, 47) are adjacent to the lens (4) of the microscope.

## Revendications

1. Microscope pour l'observation sous grand angle d'un oeil (6), avec une optique (8, 33), située entre l'objectif (4) et l'oeil (6) à traiter, projetant une image à côtés renversés, pour l'observation du fond d'oeil (9), notamment pour des opérations de l'oeil et avec un dispositif (23) insérable et pivotant, situé dans la trajectoire du faisceau (10) du microscope, pour renverser et redresser l'image, le dispositif (23) pour renverser et pour redresser l'image consistant dans un système réfléchissant de faible hauteur, un système de prismes (24) qui est porté par un support (12) fixé sur le microscope, pour être insérable et pivotant de cette manière dans la trajectoire du faisceau (10) du microscope, entre l'objectif (4) et l'oeil (6) à traiter, en ce que le système de prismes (24) se trouve directement à l'avant de l'objectif (4), à une distance par rapport à l'oeil (6), alors qu'il est prévu toutefois, entre le système de prismes (24) et l'objectif (4) une optique de reproduction (28) directement voisine de l'objectif (4) après insertion ou pivotement du système de prismes (24) dans la trajectoire du faisceau (10) du microscope, pour l'adaptation de la trajectoire du faisceau (10),dans lequel dans la trajectoire du faisceau (10), entre l'optique (8) pour observer le fond d'oeil (9) et le système de prismes (24), il est prévu une optique mobile le long de la trajectoire du faisceau (10) et par rapport au système de prismes (24), de sorte que l'optique (8) puisse être focalisée, sans être déplacée, dans lequel à l'avant et à l'arrière de chaque système de prismes (24) sont disposés chaque fois deux prismes (40 à 43) sensiblement situés dans un plan, couvrant la trajectoire du faisceau, dans lequel la base de prisme des prismes respectifs (40 à 43) situés sensiblement dans un plan sont disposées à l'opposée, dans lequel la base de prisme des prismes (42, 43) les plus proches de l'objectif sont chaque fois opposées et dans lequel celle des autres prismes (40, 41) se font face.

2. Procédé selon la revendication 1,
**caractérisé en ce que**,
l'optique (8) pour l'observation du fond d'oeil (9) est montée sur le support (12).

3. Microscope selon l'une quelconque des revendications 1 ou 2,
**caractérisé en ce que**,
le support (12) est rotatif autour d'un axe de pivotement (13) disposé sur le microscope, sur la face inférieure de ce dernier, ou est susceptible de se déplacer dans un guidage.

4. Microscope selon l'une quelconque des revendications 1 à 3,
**caractérisé en ce que**,
le système de prismes (24) est disposé dans un boîtier (25) fermé, qui est muni de jours (26, 27) pour la trajectoire du faisceau (10).

5. Procédé selon la revendication 3,
**caractérisé en ce que**,
l'axe de pivotement (13) est prévu à peu près à l'horizontale ou à la verticale sur le microscope.

6. Microscope selon l'une quelconque des revendications 1 à 5,
**caractérisé en ce que**,
l'optique (8) pour l'observation du fond d'oeil (9) est consiste dans un système de lentilles (10) disposé de façon mobile le long de la trajectoire du faisceau.

7. Microscope selon l'une quelconque des revendications 1 à 6,
**caractérisé en ce que**,
les optiques (8, 34) pour l'observation sous grand angle et/ou pour la représentation intermédiaire sont susceptibles d'être manoeuvrées au moyen d'actionneurs à broche à vis (11, 35 ; 29 à 32).

8. Microscope selon l'une quelconque des revendications 1 à 7,
**caractérisé en ce que**,
un système de prismes de Porro de 2^{ème} type ou un prisme à réflexion selon Uppendahl sert de système de prismes (24).

9. Microscope selon l'une quelconque des revendications 1 à 7,
**caractérisé en ce que**,
un prisme à réflexion selon Schmidt Pechan sert de système de prismes (24).

10. Microscope selon l'une quelconque des revendications 1 à 9,
**caractérisé en ce que**,
le support (12) est mobile le long de la trajectoire du faisceau (10) au moyen d'un premier actionneur à broche à vis (11).

11. Procédé selon la revendication 10,
**caractérisé en ce que**,
l'optique (8) est fixée sur une traverse (14) qui est guidée en déplacement longitudinal sur une tige de guidage (15) montée sur le support (12) et s'étendant à la parallèle par rapport au premier actionneur à broche à vis (11), un premier bouton rotatif (20) pour le premier actionneur à broche à vis (11) étant logé sur la broche à vis (17).

12. Procédé selon la revendication 11,
**caractérisé en ce que**,
l'optique (34) pour l'adaptation de l'observation intermédiaire est mobile le long de la trajectoire du faisceau (10) au moyen d'un deuxième actionneur à broche à vis (35) fixé sur la tige de guidage (15), la première tige de guidage (15) étant reliée par l'intermédiaire d'une patte de liaison (19) avec la deuxième tige de guidage (36) et **en ce qu'**il est prévu un deuxième bouton rotatif (40) pour l'entraînement du deuxième actionneur à broche à vis (35).

13. Procédé selon la revendication 11 ou 12,
**caractérisé en ce que**,
les boutons rotatifs (20, 40) sont à actionnement manuel.

14. Procédé selon la revendication 11 ou 12,
**caractérisé en ce que**,
au moins l'un des boutons rotatifs (20, 40) est actionnable au moyen d'un entraînement électrique (29 à 32).

15. Procédé selon la revendication 14,
**caractérisé en ce que**,
l'entraînement (29-32) comporte un moteur électrique (29) se situant de préférence sur le support (12), dont la réduction est susceptible d'être couplée en rotation avec le bouton rotatif (20, 40) par l'intermédiaire d'un arbre flexible (31).

16. Microscope selon l'une quelconque des revendications 1 à 15,
**caractérisé en ce que**,
le système de prismes (24) consiste dans deux unités de renversement et de redressement de l'image.

17. Procédé selon l'une quelconque des revendications 1 à 16,
**caractérisé en ce que**,
entre chaque prisme (40 à 43) et le système de prismes (24) est disposée une lentille convexe ou lentille divergente (44 à 47) et **en ce que** les lentilles divergentes (46, 47) sont voisines de l'objectif (4) du microscope.
